# EUROPEAN PATENT APPLICATION

(11) **EP 2 630 965 A1**
(43) Date of publication of application: **28.08.2013**
(21) Application number: 12156844.8
(22) Date of filing: 24.02.2012
(51) Int. Cl.: A61K 38/26, A61P 25/28

(54) **A polypeptide for the protection from neurodegeneration in patients with amyotrophic lateral sclerosis (ALS)**

(71) Applicant: Curatis Pharma GmbH, 30625 Hannover (DE); Medizinische Hochschule Hannover, 30625 Hannover (DE)
(72) Inventor: The designation of the inventor has not yet been filed
(74) Representative: von Kreisler Selting Werner

(57) **Abstract**

A peptide of the formula

R¹-NH-HAEGTFTSDVSSYLEGQAAKEFIAWLVK-CONR²R³

for the treatment of early stage amyotrophic lateral sclerosis (ALS), wherein R¹ = H or is an organic compound comprising from 1 to 10 carbon atoms and R²R³ = independently H or an alkyl group of 1 to 4 carbon atoms.

SEQUENCE LISTING

<110>  Curatis Pharma GmbH
       Medizinische Hochschule Hannover

<120>  A polypeptide for the protection from neurodegeneration in
       patients with amyotrophic lateral sclerosis (ALS)

<130>  113057ep

<160>  1

<170>  PatentIn version 3.3

<210>  1
<211>  28
<212>  PRT
<213>  artificial

<220>
<223>  GLP-1(7-34)amid

<400>  1

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys
            20                  25

## Description

The invention pertains to a polypeptide for the protection from neurodegeneration in patients with amyotrophic lateral sclerosis (ALS).

Amyotrophic lateral sclerosis (ALS) is a fatal neurodegenerative disease characterized by the death of motor neurons in the motor cortex, brain stem and spinal cord. There is some evidence that central nervous system (CNS) metabolic defects play a role in ALS pathogenesis.

Several different detrimental processes have been identified in neurodegenerative disorders such as ALS. ALS is the most common adult onset motor neuron disease leading to rapidly progressive degeneration of upper motor neurons in the primary motor cortex and lower motor neurons in the brain stem and spinal cord. Death usually occurs within 3-5 years after disease onset due to respiratory insufficiency [1].

The identification of mutations in the gene coding for superoxide dismutase 1 (SOD1) in familial ALS patients lead to the generation of the most commonly used and best characterized ALS animal model, the SOD1- G93A mouse which overexpresses human mutant SOD1 and closely mimics the phenotype of both familial and sporadic ALS [2, 3].

The definitive pathogenic mechanisms in ALS remain unclear. Among the factors that are thought to play an essential role are chronic excitotoxicity and metabolic disturbances affecting lipid and glucose metabolism and mitochondrial dysfunction[4].

EP -A- 1 012 188 discloses N-acetyl-GLP-1(7-34)amide and derivatives thereof for treating diabetes mellitus and obesity.

In spite of extensive research, no compound with substantial neuroprotective potential in human ALS patients has been identified yet. There is therefore an urgent need for novel therapeutic approaches in ALS.

One object of the present invention is to provide a compound for preventing progression of ALS.

According to the invention the object is accomplished by the peptide of the formula
R¹-NH-HAEGTFTSDVSSYLEGQAAKEFIAWLVK-CONR²R³
for the treatment of early stage amyotrophic lateral sclerosis (ALS), wherein R¹ = H or is an organic compound comprising from 1 to 10 carbon atoms and R²R³ = independently H or an alkyl group of 1 to 4 carbon atoms.

The present invention is based on the surprising finding that the peptide of the invention has neuroprotective effects against chronic motor neuron death. The peptide of the invention prevents progression of neurodegeneration in patients suffering from ALS and is therefore suitable for the treatment of ALS in an early stage, i.e. when pareses start in one body region and disease progression towards generalized degeneration of motor neurons can still be prevented by neuroprotective compounds.

In the sequence of the peptides of the invention the amino acids are symbolized in the single letter code, but with explicit designating the N-terminal end (R¹-NH-) and C-terminal end (-CONR²R³).

In the peptide for the use according to the invention R¹ represents an acyl group.

R¹ is in particular formyl, acetyl, propionyl, isopropionyl and/or R², R³ is independently in particular hydrogen, methyl, ethyl, propyl, isopropyl, n-butyl, sec-butyl, tert-butyl, particularly R² = R³ hydrogen, methyl or ethyl. For example the C-terminal of the peptide may be an amide, dimethylamide, diethyl amide but also mixed amides like mono methyl amide, mono ethylamide, methyl ethylamide and so on. These combinations are easily understood by the skilled person.

The peptide may be modified, wherein A in position 8 of R-GLP-1-(7-34)-amide is substituted by a neutral amino acid selected from the group, consisting of S, S†, G, C, C†, Sar, beta-ala and Aib; and/or
G in position 10 of R-GLP-1-(7-34)-amide is substituted by a neutral amino acid; and/or
D in position 15 of R-GLP-1-(7-34)-amide is substituted by an acidic amino acid.

In particular A may be substituted by S, S†, G, C, C†, Sar, beta-ala and Aib in the peptide.

The peptide may be formulated in combination with a suitable pharmaceutically acceptable carrier. Such carriers are well known to the skilled person and can be readily derived from textbooks of pharmaceutical formulation.

For example the peptide according to the invention may be formulated in a permanent or pulsative release formulation or formulated for subcutaneous, intravenous, intra-arterial, peroral, intramuscular or transpulmonary administration.

The dosage in which the peptide shall be applied is dependent on various conditions known to the medical practitioner e.g. depending on the status of the patient and the nature of the peptide and/or its formulation. For example, if the peptide is modified at its terminal ends for inducing prolonged residence times the dosage can be smaller than without the modification. In most cases the dosage can be adjusted by the physician according to his or her professional skills. Typically, the peptide can be applied in a range of from about 0.01 µg/kg body weight to 1 mg/kg body weight, in particular from about 0.05 µg/kg body weight to 30 µg/kg body weight.

### Description of the Drawings

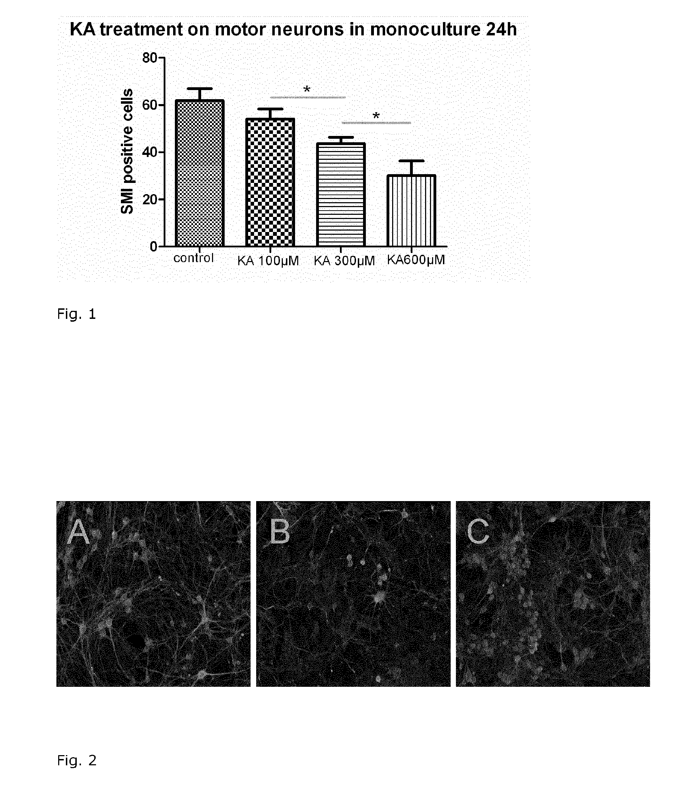

Fig.1: Dose dependent neurotoxic effects of kainic acid (KA) on wild type motor neurons cultured in monocultures as quantified by immunocytochemistry after 7 days in vitro (DIV 7) (n=6). Values represent mean ± SEM, * p < 0.05
Fig. 2: Immunocytochemical analysis of motor neuron-astrocyte co-cultures. Photographs show representative motor neuron cultures at seven days of incubation (DIV 7) on astrocyte feeder layers. A: Motor neurons without any treatment. B: Reduction of SMI 32 positive motor neurons following kainic acid (KA) exposure. C: Increase in motor neuron survival due to N-acetyl-GLP-1(7-34)amide co-incubation. Motor neurons were stained by an antibody against SMI 32 (red). Astrocytes were immunopositive for glial fibrillary acidic protein (GFAP) (green). Stained nuclei of cultured cells appear in blue (4', 6-diamidino-2-phenylindole; DAPI).

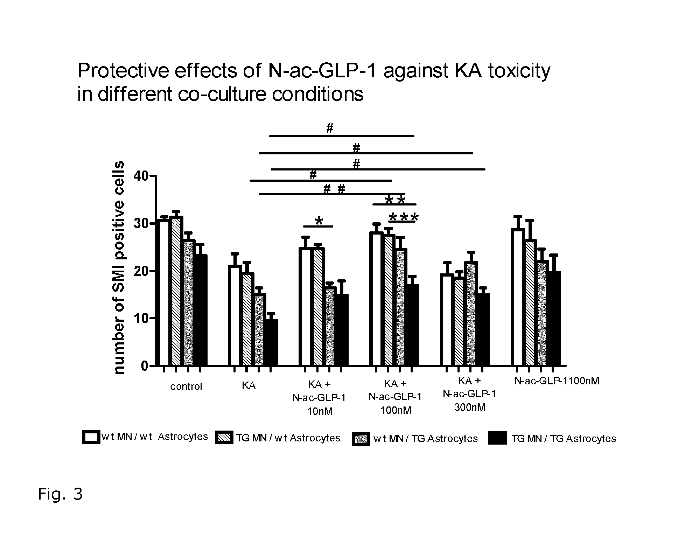

Fig.3: Assessment of the neuroprotective effect of N-acetyl-GLP-1(7-34)amide (GLP-1) in presence of kainic acid (KA). Motor neuron-astrocyte co-cultures were incubated for 24 h with 10, 100 or 300nM of N-acetyl-GLP-1(7-34)amide and 300µM KA at DIV 7 and surviving motor neurons were quantified by immunocytochemistry.

N-acetyl-GLP-1(7-34)amide protected motor neurons from toxicity induced by KA. There was no significant dose-dependent effect but the inventors observed higher numbers of surviving motor neurons (MN) following incubation with 100nM instead of 10nM of N-acetyl-GLP-1(7-34)amide. SOD1-G93A transgenic motor neurons in co-culture with SOD1-G93A transgenic astrocytes were significantly more sensitive to KA-toxicity than wild type motor neurons in co-culture with either wild type or transgenic astrocytes. The protective effect of 100nM N-acetyl-GLP-1(7-34)amide was significantly higher in the wild type MN / wild type astrocytes and in the SOD1- G93A MN/ wild type astrocytes co-cultures than in the SOD1-G93A MN / SOD1-G93A astrocytes co-culture (p< 0.05). A concentration of 300 nM N-acetyl-GLP-1(7-34)amide had neuroprotective effects only in cultures with transgenic astrocytes, but not in cultures with wild-type astrocytes. Values represent mean ± SEM, * p < 0.05. GLP-1 represents N-acetyl-GLP-1(7-34)amide.

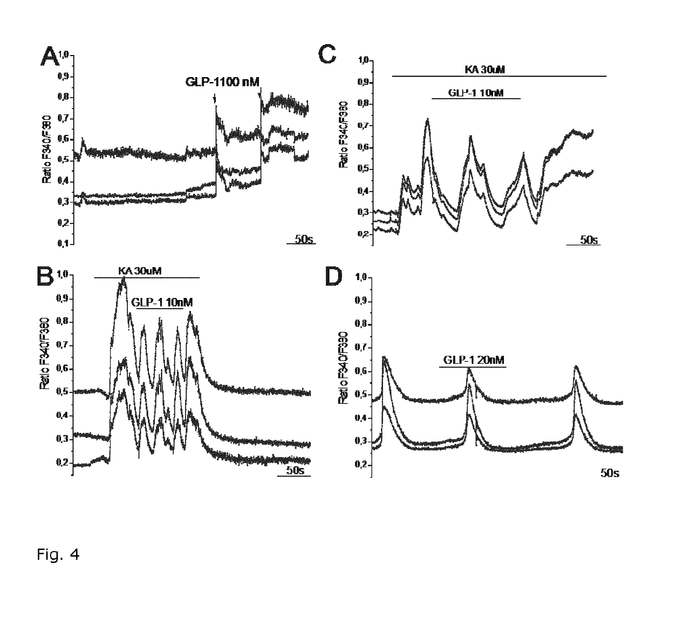

Fig.4 Microfluorometric Ca²⁺ measurements monitoring cytosolic calcium transients. Non-spontaneously active cultures (< DIV 10 , A, B,C) were exposed to long pulses of 30µM KA. Upon that, Ca²⁺ transients were observed in all region of interests (cytosol, nucleus and neurite). After 100s of KA application, 10nM N-acetyl-GLP-1(7-34)amide (GLP-1) was applied for 70s. The influx of Ca²⁺ became smaller following N-acetyl-GLP-1(7-34)amide application (B). When the application of both KA and N-acetyl-GLP-1(7-34)amide was stopped, the intracellular Ca²⁺ levels returned to baseline (B). When KA was continuously applied, all the evaluated regions of interest showed an intracellular Ca²⁺ overload when N-acetyl-GLP-1(7-34)amide application was terminated which indicates neuronal cell death, induced by the continued treatment with KA (C). Starting at DIV 10, large cells (diameter > 20µM), which were considered to be motor neurons, became spontaneously active and showed quite regular Ca²⁺ transients.

In spontaneously active cells, after 100s of spontaneous influx, a 150s pulse of 20 nM N-acetyl-GLP-1(7-34)amide was applied which also led to a slight reduction of intracellular Ca²⁺ transients (Fig. 4. D). Thus, N-acetyl-GLP-1(7-34)amide prevented the KA-induced intracellular Ca²⁺ overload and, thus, prevented neuronal cell death. Cytosolic calcium concentrations are presented as ratios of the fluorescence signals obtained at 340 and 380nm (F340/F380). GLP-1 represents N-acetyl-GLP-1(7-34)amide.

As a representative of the peptides of the invention N-acetyl-GLP-1(7-34)amide, a synthetic analogue of a natural hormone GLP-1, has been shown to protect motor neurons from kainic acid (KA)-induced degeneration in the SOD1- G93A mouse, an animal model which closely mimics the phenotype of both familial and sporadic ALS.

The general type of mechanism for the N-acetyl-GLP-1(7-34)amide effect is called neuroprotection. The cellular and molecular details of this type of biological effect are still not fully understood. While not being bound to any hypothesis or theory of modes of action, a probable mechanism of N-acetyl-GLP-1(7-34)amide action may involve activation of GLP-1 receptors on motor neurons and/or on neighbouring astrocytes and glia cells. This may in turn activate a number of signalling mechanisms inside the cells, which may prevent excitotoxicity and correct metabolic disturbances and, thus, increase the ability of motor neurons to survive.

The present invention is further illustrated by the following example which, however, is not to be construed as limiting the scope of protection. The features disclosed in the foregoing description and in the following example may, both separately and in any combination thereof, be material for realizing the invention in diverse forms thereof.

### Example

### Method.

In the present study, the inventors examined the protective effects of N-acetyl-GLP-1(7-34)amide, in mouse embryonic motor neurons, derived from both wild type and mutant SOD1-G93A transgenic mice, the most commonly used ALS animal model.

The viability of purified wild type or SOD1-G93A transgenic mouse embryonic motor neurons were investigated, cultured in a co-culture system on astrocyte feeder layers, following exposure to kainic acid (KA) as excitotoxic stimulus, in presence or absence of N-acetyl-GLP-1(7-34)amide using immunocytochemistry and calcium imaging.

### Results

In this in vitro model of excitotoxicity, SOD1-G93A transgenic motor neurons showed significantly increased vulnerability to KA as compared to wild type motor neurons. Moreover, co-culture of motor neurons together with astrocytes showed significant protective effects of wild type astrocytes on the viability of motor neurons, confirming non-cell autonomous mechanisms of motor neuron death in ALS. The inventors further detected neuroprotective effects of N-acetyl-GLP-1(7-34)amide which could be attributed to an attenuation of intracellular calcium transients. N-acetyl-GLP-1(7-34)amide therefore represents an interesting novel candidate for further in vivo evaluation in ALS.

### Materials and Methods

### Primary Culture

Isolation and in vitro cultivation of motor neurons was performed as previously described [5]. Lumbar ventral spinal cords were dissected from individual mouse embryos (gestational age: E14/15). G93A transgenic familial ALS mice (high copy number; B6SJLTg (SOD1-G93A)1Gur/J)[3] were obtained from the Jackson Laboratory (Bar Harbor, ME, USA).These mice over-express the human mutant SOD1 allele containing the Gly₉₃→Ala (G93A) substitution. The inventors maintained the transgenic G93A hemizygotes by mating transgenic males with B6SJLF1/J hybrid females. Transgenic offspring was genotyped by PCR assay of DNA obtained from the cerebellum of embryo.

After tissue dissociation, the motor neurons were enriched by a p75^{NTR}-antibody-based (P75^{NTR} antibody, Abcam, cat.no.ab27007) immunopanning technique. Culture medium (Neurobasal medium, Gibco Invitrogen, Germany; with 2% horse serum; 2% B27-supplement,Gibco Invitrogen, Germany; 0.5 mM glu-tamax-I ; 5ng/ml rHuBDNF, Peprotech, Germany and 5ng/ml rHuCNTF, Peprotech, Germany) was added to the resulting pellet. Highly enriched motor neurons were seeded on glass coverslips either pre-incubated first with polyornithin (diluted 1:1000, Sigma) and then with laminin 1(2.5µg/ml, Invitrogen) or pre-incubated first with poly-L-Lysin (diluted 1:1000, Sigma) and then plated with primary astrocytes prepared from wild type or mutant G93A transgenic neonatal mouse, as previously described [6].The average density of motor neurons was 2.0 x 10⁴ cells/cm².

### Immunocytochemistry and assessment of motor neuron survival

Cells were fixed with 4% para-formaldehyde (PFA). Glial feeder layers were stained with antibodies against glial fibrillary acidic protein (GFAP) (1:50; Sigma, Germany) to identify astrocytes. Motor neurons were stained with an antibody against SMI32 (1:100; Covance, USA) which is specific for motor neurons derived from embryonic spinal cord. The nuclei of all cultured cells were stained with 4', 6-diamidino-2-phenylindole (DAPI; Sigma). Cell counts were done in 5 visual fields of each coverslip in a total of 5-7 different preparations for each condition. Counting was performed via an ocular counting grid to quantify all cells on a horizontal stripe crossing the complete well, using a fluorescence microscope (BX-70).

### Toxicity Experiments

Motor neuron-rich cell fractions (MN) derived from either wild type or mutant G93A transgenic ALS mice (WT MN/ G93A MN) were seeded on laminin or on glial feeder layers of wild type or transgenic neonatal mouse astrocytes (WT A/G93A A) at an average density of 2.0x 10⁴ cells /cm². After 7 days in vitro (DIV 7), cell cultures were incubated for 24h with 300µM KA. Furthermore, 300µM KA was applied, together with N-acetyl-GLP-1(7-34)amide (Curatis Pharma GmbH, Germany) in different concentrations (10, 100 and 300 nM, respectively) to test for dose-dependent effects of N-acetyl-GLP-1(7-34)amide on motor neuron survival. The dose range of N-acetyl-GLP-1(7-34)amide the inventors used to examine neuroprotective properties in motor neurons was similar to the one used in experiments in neuronal-like cells as well as pancreatic β cells [7, 8]. Cells were fixed with PFA and motor neuron survival was assessed as described earlier.

### Calcium Imaging

For calcium imaging studies, all cultures were incubated for 20min at room temperature with the membrane permeable ester form of the high-affinity ratiometric calcium dye FURA 2 AM (4 µM, Sigma, Germany). Cells chosen for measurements had to exceed at minimum a diameter of 20µm, to be considered as motor neurons. Fluorescent images were obtained at temporal and spatial resolution (5Hz, TILL Vision Imaging System by Photonics, Munich, Germany). Standard extracellular solution contained HEPES 11.6 mM, Na⁺ 129.1 mM, Cl⁻ 143.8 mM, K⁺ 5.9 mM, Ma²⁺ 1.2 mM, Ca²⁺ mM, and glucose 10.0 mM at pH 7.3 (NaOH). Imaging experiments were conducted at room temperature and after obtaining baseline values, local perfusion through the applicator was started using long 170s pulses of 30µM KA and long 75s pulses of 10nM N-acetyl-GLP-1(7-34)amide. For the analysis of Ca²⁺ transients, background subtraction was used and subcellular regions of interest were defined over the cytosol, nucleus and neurite.

### Statistical Analysis

All results were expressed as mean ± SEM. GraphPad Prism 3.0 software was used for statistical evaluation. Comparisons between different conditions were performed using Mann- Whitney test.

### Results

Dose-dependent neurotoxic effects of kainite (KA) on primary motor neurons To induce excitotoxicity, 7-day-old (DIV 7) wild type motor neuron monocultures were exposed to KA in concentrations ranging from 100 to 600 µM for 24h (Fig.1) and a dose-dependent effect was observed, as previously described [9, 10]. In light of this result, 300 µM KA was chosen for further experiments.

### SOD1-G93A transgenic motor neurons are more sensitive to KA-induced excitotoxicity and wild type astrocytes have protective effects

In motor neuron-astrocyte co-cultures, there were larger neuronal networks when compared with monocultures. Motor neurons in 7-day old (DIV 7) cultures were all SMI 32 positive and had cell bodies larger than 20µM and a distinct morphology (Fig.2). Astrocytes were identified by GFAP-immunostaining [10]. Motor neuron survival in the four different co-culture conditions was examined by counting the proportion of SMI 32-positive motor neurons following double-immunostaining for β-tubulin. Under KA exposure (300µM), there was a significantly increased sensibility of G93A transgenic motor neurons compared to the wild type ones (p<0.05). When comparing the co-culture of G93A MN/WT A (19.37 ± 2.13%) and G93A MN/G93A A (8.25 ± 1.03%), even without addition of N-ac-GLP-1, larger numbers of transgenic motor neurons in G93A MN/WT A system than of those co-cultured with G93A astrocytes were counted (p<0.05). In wild type motor neurons, on the other hand, the inventors also observed a larger reduction of cell counts following KA exposure when they were surrounded by SOD1-G93A instead of wild type astrocytes (p<0.05) (Fig. 3). This indicates a protective effect of wild type astrocytes on G93A transgenic motor neurons and some toxic effects of transgenic astrocytes.

N-acetyl-GLP-1(7-34)amide has neuroprotective effects on wild type and SOD1-G93A transgenic motor neurons in our in vitro model of KA-induced excitotoxicity.

To assess the neuroprotective effects of N-acetyl-GLP-1(7-34)amide in our co-culture system, 300 µM KA was applied together with N-acetyl-GLP-1(7-34)amide. Dose-dependent effects of N-acetyl-GLP-1(7-34)amide on developing spinal motor neurons under the four different co-culture conditions were assessed, using three different concentrations of N-acetyl-GLP-1(7-34)amide (10, 100, 300 nM) starting at DIV 7 for 24h.

Neuroprotective effects of N-acetyl-GLP-1(7-34)amide against KA-toxicity were observed in both wild type and transgenic motor neurons (Fig. 3). The neuroprotective effect of 100nM N-acetyl-GLP-1(7-34)amide did differ significantly between both WT MN/WT A (28.05 ± 2.39%) or G93A MN/WT A (26.84 ± 1.68%) and G93A MN/G93A A (15.05± 1.09% , p<0.05)(Fig.3), with most dramatic neuroprotective effects seen in the combination of 100nM N-acetyl-GLP-1(7-34)amide applied on transgenic motor neurons surrounded by wild type astrocytes. When comparing WT MN/WT A and WT MN/ G93A, the difference of neuroprotective effect of 10nM N-acetyl-GLP-1(7-34)amide was also significant (p<0.05).

Between 10 and 100nM N-acetyl-GLP-1(7-34)amide, there was an increase in the number of surviving motor neurons in all four co-culture conditions while a concentration of 300 nM N-acetyl-GLP-1(7-34)amide had neuroprotective effects only in cultures with transgenic astrocytes, but not in cultures with wild-type astrocytes (Fig.2, fig.3).

### N-acetyl-GLP-1(7-34)amide reduced KA-induced and spontaneously occurring intracellular calcium transients in motor neurons

To further analyse the mechanism underlying the neuroprotective effects of N-acetyl-GLP-1(7-34)amide against KA - excitotoxicity at the physiological level, calcium imaging experiments were performed (Fig.4). Motor neurons in monoculture younger than DIV 10 were observed to be non-spontaneously active, i.e. they did not show occurrence of spontaneous intracellular Ca²⁺ transients.

A 170s pulse of 30µM KA induced Ca²⁺ transients in motor neurons < DIV10. After 100s of KA application, 10nM N-acetyl-GLP-1(7-34)amide was applied for 70s.This led to a decrease of the Ca²⁺ influx which then returned to baseline when the application of N-acetyl-GLP-1(7-34)amide together with KA was stopped. (Fig.4. B). Under continued KA exposure after termination of N-acetyl-GLP-1(7-34)amide application all cells showed an intracellular Ca²⁺ overload which indicates neuronal cell death (Fig. 4. C). Thus, N-acetyl-GLP-1(7-34)amide prevented the KA-induced intracellular Ca²⁺ overload and, thus, prevented cell death.

Starting at DIV 10, large cells (diameter > 20µM), which were considered to be motor neurons, were spontaneously active and showed quite regular Ca²⁺ transients. In spontaneously active cells, after 100s of spontaneous influx, a 150s pulse of 20 nM N-acetyl-GLP-1(7-34)amide was applied which also led to reduction of intracellular Ca²⁺ transients (Fig. 4. D).

### References

1. Bruijn, L.I., Miller, T.M., Cleveland, D.W., Unravelling the mechanisms involved in neuron degeneration in ALS. Annu Rev Neurosci, 2004. 27: p. 723-49.
2. Rosen, D.R., A shared chromosome-21 haplotype among amyotrophic lateral sclerosis families with the A4V SOD1 mutation. Clin Genet, 2004. 66(3): p. 247-50.
3. Gurney, M.E., et al., Motor neuron degeneration in mice that express a human Cu,Zn superoxide dismutase mutation. Science, 1994. 264(5166): p. 1772-5.
4. Pasinelli, P. and R.H. Brown, Molecular biology of amyotrophic lateral sclerosis: insights from genetics. Nat Rev Neurosci, 2006. 7(9): p. 710-23.
5. Wiese, S., et al., Isolation and enrichment of embryonic mouse motoneurons from the lumbar spinal cord of individual mouse embryos. Nat Protoc. 5(1): p. 31-8.
6. Stangel, M. and D. Bernard, Polyclonal IgM influence oligodendrocyte precursor cells in mixed glial cell cultures: implications for remyelination. J Neuroimmunol, 2003. 138(1-2): p. 25-30.
7. Doyle, M.E. and J.M. Egan, Glucagon-like peptide-1. Recent Prog Horm Res, 2001. 56: p. 377-99.
8. Perry, T., et al., Protection and reversal of excitotoxic neuronal damage by glucagon-like peptide-1 and exendin-4. J Pharmacol Exp Ther, 2002. 302(3): p. 881-8.
9. Ragancokova, D., et al., Analysis of neuroprotective effects of valproic acid on primary motor neurons in monoculture or co-cultures with astrocytes or Schwann cells. Cell Mol Neurobiol, 2009. 29(6-7): p. 1037-43.
10. Vandenberghe, W., L. Van Den Bosch, and W. Robberecht, Glial cells potentiate kainate-induced neuronal death in a motoneuron-enriched spinal coculture system. Brain Res, 1998. 807(1-2): p. 1-10.

## Claims

1. A peptide of the formula
R¹-NH-HAEGTFTSDVSSYLEGQAAKEFIAWLVK-CONR²R³
for the treatment of early stage amyotrophic lateral sclerosis (ALS),
wherein R¹ = H or is an organic compound comprising from 1 to 10 carbon atoms and R²R³ = independently H or an alkyl group of 1 to 4 carbon atoms.

2. The peptide of claim 1, wherein R¹ represents an acyl group.

3. The peptide of claim 1 or 2, wherein R¹ is formyl, acetyl, propionyl, isopropionyl and/or R², R³ is independently hydrogen, methyl, ethyl, propyl, isopropyl, n-butyl, sec-butyl, tert-butyl, in particular R² = R³ hydrogen, methyl or ethyl.

4. The peptide according to at least one of the claims 1-3, wherein
A in position 8 of R-GLP-1-(7-34)-amide is substituted by a neutral amino acid selected from the group, consisting of S, S†, G, C, C†, Sar, beta-ala and Aib; and/or
G in position 10 of R-GLP-1-(7-34)-amide is substituted by a neutral amino acid; and/or
D in position 15 of R-GLP-1-(7-34)-amide is substituted by an acidic amino acid.

5. The peptide according to claim 4 wherein A is substituted by S, S†, G, C, C†, Sar, beta-ala and Aib.

6. The peptide of at least one of the claims 1 to 5 in combination with a suitable pharmaceutically acceptable carrier.

7. The peptide according at least one of the claims 1 to 6 **characterized in that** said peptide is formulated in a permanent or pulsative release.

8. The peptide according at least one of the claims 1 to 6 **characterized in that** said peptide is formulated for subcutaneous, intravenous, intraarterial, peroral, intramuscular or transpulmonary administration.

9. The peptide according at least one of the claims 1 to 8 **characterized in that** said peptide is administered in a dosage range of from about 0.01 µg/kg body weight to 1 mg/kg body weight, in particular from about 0.05 µg/mg/kg body weight to 30 µg//kg body weight.
